# EUROPÄISCHE PATENTSCHRIFT

(11) **EP 1 236 444 B1**
(45) Veröffentlichungstag und Bekanntmachung des Hinweises auf die Patenterteilung: **02.08.2006**
(21) Anmeldenummer: 02003380.9
(22) Anmeldetag: 14.02.2002
(51) Int. Cl.: A61C 19/00

(54) **Einrichtung zur Polymerisation von lichthärtenden Kunststoffen, insbesondere von zahnärztlichen Füll-oder Klebematerialien**
Device for the hardening of light-curing resins, especially of dental fill- and adhesive materials
Dispositif pour la polymérisation de résines, en particulier pour des materiaux de remplissage et de collage dentaires

(30) Priorität: 14.02.2001 DE 10107099
(43) Veröffentlichungstag der Anmeldung: 04.09.2002
(73) Patentinhaber: Sirona Dental Systems GmbH, 64625 Bensheim (DE)
(72) Erfinder: Kratochwilla, Hans Michael, 64653 Lorsch (DE)
(74) Vertreter: Sommer, Peter

(56) Entgegenhaltungen:
- EP-A- 0 750 889
- EP-A- 0 993 810
- US-A- 5 040 964

## Beschreibung

Die Erfindung bezieht sich auf eine Einrichtung zur Polymerisation von lichthärtenden Kunststoffen, insbesondere von zahnärztlichen Füll- oder Klebematerialien, entsprechend dem Oberbegriff des Patentanspruches 1.
Derartige Polymerisationseinrichtungen dienen dazu, einen photoreaktiven Aushärteprozess mit Licht, welches einen hohen Blauanteil hat, auszulösen. Im Dentalbereich kann man damit in situ eingebrachte Zahnfüllungen aus Kunststoff oder Klebstoffe mit denen beispielsweise keramische Füllungen in eine Zahnkavität eingesetzt werden, aushärten. Eine weitere Anwendung wird in der Beschleunigung von chemischen Reaktionen bei auf den Zahnschmelz äußerlich aufgetragenen Aufhellsubstanzen durch intensive Lichtbestrahlung gesehen.
Die heute am Markt befindlichen Polymerisationseinrichtungen haben meist die Form eines Handstückes. Als Lichtquelle werden Halogenlampen oder Xenon-Lichtbogenlampen verwendet. Auch die Verwendung blauer Leuchtdioden (In-GaN) sind bekannt. Eine elektronische Steuereinheit bestimmt die Ausgangsleistung des Aushärtelichtes sowie die Ein- und Ausschaltzeiten.
Die Lichtleitsysteme mit denen das Polymerisationslicht von der Lichtquelle zum behandelnden Objekt geleitet wird, enthalten meist einen Lichtleiter der aus einer Vielzahl von Glasfasern besteht, die zu einem Faserstab gebündelt sind. Das Polymerisationslicht tritt am freien Ende des Lichtleiters an einem entsprechend ausgebildeten Lichtaustrittsfenster aus. Um eine Aushärtung bei vertretbar niedriger Leistung der Lichtquelle zu erzielen, muß das Lichtaustrittsfenster möglichst nahe (1 mm oder näher) an die betreffende Klebe- oder Aushärtestelle gebracht werden. Das Lichtaustrittsfenster darf dabei aber nicht mit der Bearbeitungsstelle in Berührung kommen, da ansonsten das Lichtaustrittsfenster des Lichtleiters durch unausgehärteten Kleber verkleben und damit unbrauchbar gemacht werden würde.
In der EP 0993 810 A2 wird eine Polymerisationslampe zum Aushärten von Kunststoffüllungen beschrieben, bei der der Polymerisationslampe eine Steuereinheit zugeordnet ist welche die Einschaltdauer und die Lichtintensität der Polymerisationslampe steuert und zwar so, dass Einschaltdauer und Lichtintensität nach bestimmten Betriebsparametern, entsprechend einer vorgegebenen Kurve ablaufen. Betriebsparameter sind einerseits der verwendete Werkstoff, d.h. die auszuhärtende Paste, und andererseits der Abstand zwischen Paste und der Lichtaustrittsstelle beim Aushärten. Der Abstand wird mittels eines Referenzlichts gemessen und mit vorgegebenen Werten, die in einem Speicher abgelegt sind, verglichen. Aus dem Vergleich werden die notwendigen Werte für die abzugebende Leistung und die Aushärtezeit errechnet mit denen dann die Polymerisationslampe entsprechend der vorgegebenen Kurve betrieben wird.
Das Dokument liefert keinen Hinweis, wie und wann das Einschalten der Polymerisationslampe von statten gehen soll. Demnach muß angenommen werden, dass das Einschalten in der gewohnten Weise durch einen manuell oder per Fuß zu betätigender Schalter erfolgt. Dies bedeutet aber, dass die Polymerisationslampe zu jedem Zeitpunkt, also auch vorzeitig, ob bewußt oder unbewußt, eingeschaltet werden kann.
In EP 0 780 103 A2 wird ein Bestrahlungsgerät zur Aushärtung von Kunststoffen beschrieben, bei dem als Lichtquelle ein oder mehrere lichtemittierende Dioden (LEDs) vorgesehen sind. Die Dioden arbeiten im Bereich von etwa 320 bis 550 nm. Die Lichtintensität kann mittels eines Sensors gemessen werden. Bei Verwendung mehrerer Dioden sind die Dioden in einer gemeinsamen Kapselung angeordnet, die in Lichtaustrittsrichtung als Linse ausgebildet ist. Der Betriebsstrom der Dioden kann zur Erzielung einer höheren Lichtintensität in einem bestimmten Pulsverhältnis (Impuls/Pause) gepulst werden.
Bei der Handhabung der bekannten Polymerisationseinrichtungen kann es leicht zu einem vorzeitigen, ungewollten Aktivieren des Lichtes und damit zu einem vorzeitigen Auslösen des Aushärtevorganges kommen. Beispielsweise kann es während des Suchens des Auslöseschalters am Fußschalter und/oder beim Positionieren des Handstückes in Bezug zur Aushärtestelle zu einem vorzeitigen Einschalten kommen, wodurch nicht nur ungewollt und unkontrolliert Licht auf die betreffende Objektstelle geleitet wird, sondern auch die Gefahr einer Berührung der Kleb- oder Aushärtestelle mit dem Lichtaustrittsfenster besteht.
Das vorzeitige Starten des Aushärtelichts hat verschiedene Nachteile und birgt auch gewisse Risiken mit sich. So ist die in der Regel von einem Timer vorgegebene Zeitbasis bei einem vorzeitigen Einschalten dann nicht mehr korrekt. Das belichtete photoreaktive Material ist dann nicht ausreichend aus- bzw. durchgehärtet. Weiterhin kann es zu unangenehmen Blendungen des Patienten kommen wenn das Lichtaustrittsfenster noch nicht auf die Aushärtestelle positioniert ist. Bei Anwendung von Lichtbogenlampen als Lichterzeuger sind bei solchen Blendungen eine Schädigung der Augen nicht auszuschließen.
Der im Patentanspruch 1 angegebenen Erfindung liegt die Aufgabe zugrunde, demgegenüber eine Verbesserung zu erzielen und eine Einschaltvorrichtung für zahnärztliche Polymerisationslampen der vorgenannten Gattung anzugeben, der die vorgenannten Nachteile nicht anhaften.
Die Erfindung wird nachfolgend anhand der Zeichnung näher erläutert. Es zeigen:
Figur 1 eine Polymerisationseinrichtung nach der Erfindung in einer Prinzipdarstellung, mit einer ersten Variante eines optischen Systems zur Lichtführung,
Figur 2 einen Messtubus zur Funktionsüberprüfung des optischen Systems in schaubildlicher Darstellung,
Figur 3 eine zweite Variante eines optischen Systems,
Figur 4 eine dritte Variante eines optischen Systems,
Figuren 5 und 6 eine vierte Variante eines optischen Systems,
Figur 7 eine fünfte Variante eines optischen Systems.

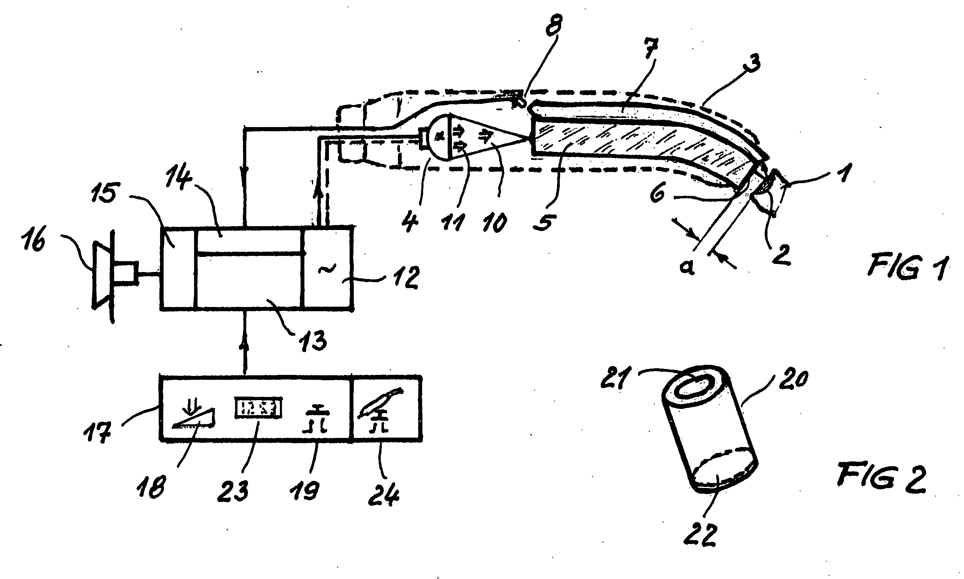

Die Figur 1 zeigt in einer Prinzipdarstellung die erfindungsgemäße Einrichtung in Verbindung mit einem typischen Anwendungsfall, dem Aushärten einer Zahnfüllung.

Die Kavität eines mit 1 angedeuteten Zahnes ist mit einem lichtaushärtenden Kunststoffmaterial 2, nachfolgend mit Objekt bezeichnet, gefüllt. Das Aushärten erfolgt mittels eines in der Darstellung gestrichelt angedeuteten Handstückes 3, in welchem eine Lichtquelle 4 in Form einer mit Reflektor ausgestattete Halogenlampe angeordnet ist. Das vom Reflektor der Halogenlampe gebündelte Licht wird im Zentrum des einen stirnseitigen Endes eines Lichtleiters 5 eingespeist und tritt am anderen, freien Ende des Lichtleiters an einem Lichtaustrittsfenster 6 aus. Der Lichtleiter 5 besteht aus einem gebogenem Glasfaserstab, der in geeigneter Weise im Handstück gehaltert ist.
Entlang der Peripherie des Lichtleiters 5 ist ein weiterer Lichtleiter 7 angeordnet, über den am Objekt 2 reflektiertes Licht zu einem im Handstück 3 entsprechend gehalterten Lichtsensor 8 (beispielsweise einer Photodiode) geleitet wird.
Die Lichtquelle 4 ist im vorliegenden Anwendungsfall so ausgeführt und geschaltet, dass einerseits Licht mit relativ geringer Energie erzeugt und dieses Licht als Pilotlicht eingesetzt werden kann, und dass andererseits bei voller Leistung der Lichtquelle Polymerisationslicht, also Licht mit zum Aushärten des Kunststoffmaterials 2 geeignetem hohen Blauanteil, erzeugt werden kann. Je nach Beschaltung kann somit einerseits Pilotlicht, angedeutet durch den einen Pfeil 10, oder Polymerisationslicht, angedeutet durch die beiden Pfeile 11, erzeugt werden.
Das Pilotlicht 10 wird durch denselben Lichtleiter 5 geleitet wie das Polymerisationslicht 11. Das Pilotlicht 10 tritt am Lichtaustrittsfenster 6 aus und wird am Objekt 2 reflektiert. Die reflektierten Strahlen werden über den Lichtleiter 7 zum Lichtsensor 8 geleitet.
Das Lichtaustrittsfenster 6 am Ende des Lichtleiters hat vorteilhafterweise einen kreisförmigen Querschnitt von etwa 3 bis 10 mm Durchmesser.
Die Lichtquelle 4 wird von einer extern des Handstückes angeordneten Energieversorgungsquelle 12 gespeist, die mit einer Steuerelektronik 13 verbunden ist.
Die Steuerelektronik 13 enthält eine nicht näher bezeichnete Auswerteelektronik in der die vom Lichtsensor 8 abgegebenen und in einem Verstärker 14 verstärkten Signale verarbeitet werden. Die Steuerelektronik 13 arbeitet mit einem Timer 15 zusammen in welchem ein oder mehrere Aushärtezeiten gespeichert sind. Der Timer 15 aktiviert nach Ablauf der Aushärtezeit einen Signalgeber 16, im dargestellten Anwendungsfall einen akustischen Signalgeber.
Mit 17 ist eine Bedieneinheit bezeichnet, die ein Display 23, einen Fußschalter 18, einen von Hand betätigbares Schaltelement 19 oder einen in einer Ablagevorrichtung des Handstückes 3 angeordneten Schalter 24 enthalten kann. Über diese Schaltelemente kann der Aushärteprozess in Gang gebracht werden.
Bei Betätigung des Fuß- oder Handschalters 18 bzw. 19 oder bei Aktivierung des Handstück-Ablageschalters 24 wird die Steuerelektronik 13 dazu veranlaßt, zunächst das Pilotlicht 10 einzuschalten. Das Pilotlicht kann dann kontinuierlich leuchten; mit Vorteil kann es aber auch periodisch getaktet werden, beispielsweise im Takt von einer oder mehrerer Sekunden. Auch eine höhere Taktfrequenz ist denkbar.

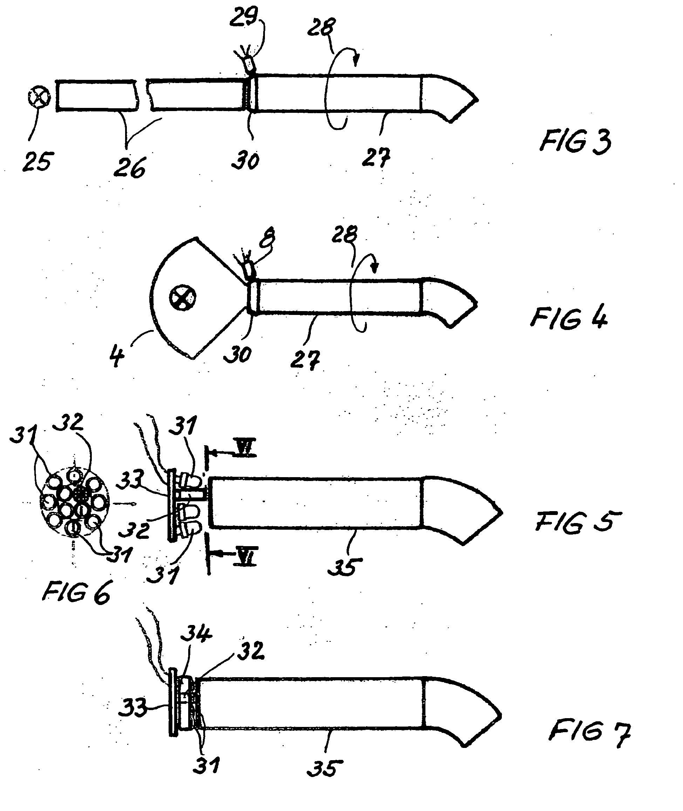

Das Pilotlicht 10 wird in der dargestellten Ausführung über den gesamten Querschnitt des Glasstabes geleitet; alternativ kann es auch über einen eigenen Lichtleiterstrang zum Lichtaustrittsfenster 6 geführt werden. In einem solchen Fall ist es vorteilhaft, als Lichtleiter ein Glasfaserbündel zu verwenden und einen Teil des Glasfaserbündels ausschließlich zur Übertragung des Pilotlichtes einzusetzen.
Zur Rückleitung des reflektierten Lichtes bis zum Lichtsensor 8 sind verschiedene Ausführungen denkbar. So kann auch dafür ein Teil des Glasfaserstabes 5 herangezogen werden, indem dieser am einen Ende konisch angeschliffen wird (Figuren 3 und 4). Ebenso können einzelne Faserstränge aus dem Lichtfaserbündel abgezweigt werden (Figuren 5 und 6). Auch andere, hier nicht gezeigte optische Übertragungsmittel sind denkbar um das reflektierte Licht in den Lichtsensor 8 einzukoppeln ohne dass der Querschnitt des Lichtleiters zur Übertragung des Polymerisationslichtes nennenswert geschmälert werden würde.
Das Ausgangssignal des Lichtsensors 8 wird im Verstärker 6 verstärkt und an die Steuerelektronik 13 gegeben die ein Startsignal zum Einschalten bzw. Zuschalten des Polymerisationslichtes 11 und des Timers 15 gibt.
Erfindungsgemäß ist die Ansprechschwelle des Verstärkers 14 so eingestellt, dass der Lichterzeuger für das Polymerisationslicht erst bei Annäherung auf einen individuell zu definierenden Abstand (a) an das Objekt 2 anspricht, z. B. bei Erreichen eines Abstandes von 1 mm. Die Ansprechschwelle ist vorteilhafterweise variabel einstellbar ausgeführt und kann so der Arbeitsweise der Bedienperson angepaßt werden. So kann anstelle eines gewünschten Abstandes von 1 mm für den einen Benutzer ein Abstand von 3 mm oder 5 mm für einen anderen Benutzer eingestellt werden.
Um Fehlschaltungen durch Fremdlichteinfall weitgehend auszuschalten, ist das vom Lichtsensor 8 zu empfangende Licht in seinem Spektrum durch geeignete Maßnahmen auf vorzugsweise 400 bis 500 nm zu begrenzen. Diese können durch Vorschalten eines Filters oder durch Verwendung einer bereits auf die genannte spektrale Empfindlichkeit abgestimmten Photodiode realisiert sein.
Gemäß einer besonders vorteilhaften Ausführung der Erfindung wird der Lichterzeuger der das Polymerisationslicht 11 erzeugt, während des Aushärtevorganges periodisch kurzzeitig abgeschaltet und in den Abschaltphasen das Pilotlicht 10 ein- oder zugeschaltet. Das kurze Abschalten kann im Bereich vom einigen Millisekunden bis max. 100 Millisekunden im Zeitabstand von einer bis weniger Sekunden geschehen. Durch diese Maßnahme kann ständig der Abstand (a) des Lichtaustrittsfensters 6 von Objekt. 2 detektiert werden. Die Einrichtung kann so automatisch erkennen, ob der richtigen Abstand eingehalten wird, der Aushärteprozess beendet ist oder dieser durch einen nicht ausreichenden Abstand unterbrochen worden ist. Durch Vergleich der bereits abgelaufenen Zeit mit der im Timer festgelegte Aushärtezeit kann somit die noch nachzuholende Aushärtezeit ermittelt und gegebenenfalls angezeigt werden.
Bei kurzen Unterbrechungen des Aushärteprozesses kann der Timer angehalten werden. Bei erneuter Annäherung des Handstückes kann der Timer dann wieder aktiviert werden wenn das Lichtaustrittsfenster im vorgeschriebenen Abstand (a) zum Objekt 2 gehalten wird.
Das Zurücksetzen des Timers auf Null würde dann erst durch Überschreiten diese Zeitbereiches oder bei Ablegen des Handstückes in eine Ablage mit entsprechender Abschaltung erfolgen.
Das beschriebene automatische Erkennen und Abschalten des Lichtaushärteprozesses kann auch mit der üblichen Hand- oder Fußsteuerung kombiniert werden, in dem bei Betätigung der manuellen Starttaste 19 die Automatik überbrückt und über eine Stoppbetätigung wieder scharf geschaltet wird.
Gemäß einer weiteren vorteilhaften Ausgestaltung der erfindungsgemäßen Einrichtung ist dem einen Ende des Lichtleiters 5 der das Lichtaustrittsfenster enthält, ein Messtubus 20 (Figur 2) zugeordnet, mit dem das optische System bzw. die Leistungsfähigkeit des Systems überprüft werden kann.
Der Messtubus 20 weist eine innere Mantelfläche 21 auf die das Licht total absorbiert und eine Stirnfläche 22 die das Licht total reflektiert, wobei der Reflexionsgrad dieser Stirnfläche geeicht ist. Zur Überprüfung des Systems wird der Messtubus 20 mit definiertem Anschlag auf das Lichtleiterende aufgesetzt. Das Lichtaustrittsfenster 6 hat dann einen genau festgelegten Abstand zur Stirnfläche 22 des Messtubus. Bei stabilisierter Auswerteelektronik und Verwendung eines kalibrierten Lichtsensors kann das Ausgangssignal der Auswerteelektronik ein Messwerk ansteuern welches dem Benutzer anzeigt, dass das System eine ausreichende Leistung zur Verfügung stellt. Insbesondere alterungsbedingte Defekte im optischen System können so leicht erkannt und gegebenenfalls durch eine Leistungsanpassung der Lichtquelle ausgeglichen werden. Der Prüfbefund kann im Display 23 des Bedienteils 17 (Figur 1 ) angezeigt werden.
In den Figuren 3 bis 7 sind verschiedene Ausführungen einer Lichteinkoppelung in einen Lichtleiter sowie Möglichkeiten einer Detektion des reflektierten Lichtes am Lichtsensor dargestellt, wobei zur Vereinfachung nur die prinzipielle Anordnung der relevanten lichttechnischen Bauteile aufgezeigt ist.
Bei der Version nach Figur 3 ist angenommen, dass ein Lichterzeuger 25 vorgesehen ist der nicht im Handstück selbst angeordnet ist, wie bei der Ausführung nach Figur 1; vielmehr ist der Lichterzeuger dort extern in einem separaten (hier nicht gezeigten) Versorgungsteil. Das Licht wird zunächst von diesem Versorgungsteil über einen flexiblen Lichtleiter 26 zum Handstück und dort in einen abgekröpften Glasstab 27 eingespeist. Der Glasstab 27 ist, wie durch den Pfeil 28 angedeutet, im (nicht dargestellten) Handstück drehbar angeordnet und zu Reinigungszwecken auch abnehmbar gehaltert.
Der Glasstab 27 besteht üblicherweise aus einer Vielzahl von dünnen Einzelfasern. Im Bereich des Lichtsensors 29 (vergleichbar mit dem Lichtsensor 8 in Figur 1) ist eine an der äußeren Mantelfläche befindliche Lage des Faserbündels kegelförmig angeschliffen und poliert. Die Anordnung ist dabei so getroffen, dass das vom Objekt zurückgeworfene Licht etwa senkrecht zur Mantelfläche abgestrahlt wird. Der Lichtsensor ist unmittelbar korrespondierend zu dieser Abstrahlfläche angeordnet. Der Glasstab bzw. dasjenige Teil des Handstückes welches den Glasstab aufnimmt, kann so um seine Längsachse beliebig gedreht werden, wobei in jeder Drehstellung eine annähernd gleiche Lichtmenge auf den Lichtsensor 29 fällt.
Die Figur 4 zeigt eine Version, bei der ein solcher drehbarer Glasstab, entsprechend der Handstückanordnung nach Figur 1, also mit im Handstück angeordneter Lichtquelle 4, ausgeführt ist.
Die Figuren 5 und 6 zeigen eine Version, bei der als Lichterzeuger und als Lichtsensor mehrere Dioden 31, 32 vorgesehen sind die auf einem gemeinsamen Träger 33 angeordnet sind. Die das Polymerisationslicht und Pilotlicht erzeugenden Dioden 31 sind Hochleistungsleuchtdioden die blaues Licht abgeben; die Diode 32 ist als Empfänger-Diode ausgebildet.
Die Anzahl der eingesetzten Leuchtdioden richtet sich nach gewünschter Lichtleistung und Ausführung der gewählten Dioden. Denkbar ist es, auf einem gemeinsamen Träger 50 und mehr Dioden anzuordnen.
Zur Fixierung der Sende- und Empfangsdioden 31, 32 können diese vorteilhafterweise mit einem gemeinsamen Linsenkörper 34 umspritzt sein der das Licht auf die Einspeisestelle in den Glasstab 35 fokussiert (Figur 7). Ein solches Element kann bezogen auf eine Abstrahlfläche von der Größe des Lichteintrittsfensters des Glasstabes mit größerer Lichtausbeute hergestellt werden.
Die Ausführungen nach den Figuren 5 bis 7 haben gegenüber den anderen Ausführungen den Vorteil eines sehr Platz sparenden Aufbaus. Weiterhin kann ein Glassstab 35ohne zusätzliche Bearbeitung eingesetzt werden. Die Dioden können außerdem sehr leicht einzeln oder in Gruppen beschaltet und damit auch als Pilotlicht eingesetzt zu werden.
Bei der kompakten Anordnung kann die Energieversorgungsquelle sehr klein dimensioniert und deshalb sogar im Handstück, beispielsweise im rückwärtigen Teil des Handstückes in Form einer Batterie oder eines Akkus angeordnet sein.
Die vorbeschriebene Einrichtung hat noch den weiteren Vorteil, dass die Lebensdauer der eingesetzten Lichterzeuger erheblich verlängert werden kann, weil der Lichterzeuger nur solange wie zur Aushärtung notwendig eingeschaltet ist.

### Bezugszeichenliste

- 1: = Zahn
- 2: = Füllmaterial
- 3: = Handstück
- 4: = Lichtquelle
- 5: = Lichtleiter
- 6: = Lichtaustrittsfenster
- 7: = Lichtleiter
- 8: = Lichtsensor
- 9: =
- 10: = Pilotlicht
- 11: = Polymerisationslicht
- 12: = Energieversorgungsquelle
- 13: = Steuerelektronik
- 14: = Verstärker
- 15: = Timer
- 16: = Signalgeber
- 17: = Bedieneinheit
- 18: = Fußschalter
- 19: = Schaltelement
- 20: = Meßtubus
- 21: = Mantelfläche
- 22: = Stirnfläche
- 23: = Display
- 24: = Handstück-Ablageschalter
- 25: = Lichtquelle
- 26: = Lichtleiter
- 27: = Glasstab
- 28: = Pfeil
- 29: = Lichtsensor
- 30: = Abstrahlfläche
- 31: = Leuchtdioden
- 32: = Empfängerdiode
- 33: = Träger
- 34: = Linsenkörper
- 35: = Glasstab

## Patentansprüche

1. Einrichtung zur Polymerisation von lichthärtenden Kunststoffen, insbesondere von zahnärztlichen Füll- oder Klebematerialien, enthaltend mindestens eine Lichtquelle (4, 25, 31,) zur Erzeugung einerseits eines Polymerisationslichtes (11) in einem zur Polymerisation geeigneten Lichtspektrum und andererseits eines Pilotlichtes (10), eine Energieversorgungsquelle (12) zur Speisung der Lichtquelle, ein sich an die Lichtquelle anschließendes, Lichtleitsystem mit Lichtleitern (5, 7, 26, 27, 35) einerseits zur Übertragung des Polymerisationslichtes (11) und des Pilotlichtes (10) auf das zu bearbeitende Objekt (2), und andererseits zur Einkopplung des am Objekt (2) reflektierten Pilotlichtes (10) in einen Lichtsensor (8, 29, 32), enthaltend ferner eine elektronische Steuereinheit (13) in welcher die vom Lichtsensor (8, 29, 32) ausgehenden Signale verarbeitet werden und welche der Lichtquelle (4, 25, 31) für das Polymerisationslicht (11) und einem die Einschaltdauer der Lichtquelle bestimmenden Timer (15) ein Startsignal geben, **dadurch** ge- kennzeichnet, dass ein Verstärker (14) vorgesehen ist der die vom Lichtsensor (8, 29, 32) ausgehenden Signale verstärkt und dass die Ansprechschwelle des Verstärkers (14) so eingestellt ist, dass die Lichtquelle für das Polymerisationslicht (11) erst bei Erreichen eines definierten Abstandes (a) eingeschaltet wird.

2. Einrichtung nach Patentanspruch 1,**dadurch gekennzeichnet, dass** Lichtquelle (4, 25, 31) für das Polymerisationslicht (11) periodisch ein- und abgeschaltet wird und in den Abschaltphasen das Pilotlicht (10) eingeschaltet wird, wobei die Einschaltphasen für das Pilotlicht im Vergleich zu den Einschaltphasen des Polymerisationslichtes sehr kurz sind.

3. Einrichtung nach Patentanspruch 2, **dadurch gekennzeichnet, dass** in den Abschaltphasen des Polymerisationslichtes (11) der Timer (15) angehalten und erst bei Erreichen des Abstandes (a) wieder aktiviert wird.

4. Einrichtung nach Patentanspruch 1, **dadurch gekennzeichnet , dass** das Pilotlicht (10) während einer Objektbearbeitung kontinuierlich eingeschaltet ist.

5. Einrichtung nach einem der Patentansprüche 1 bis 4,
**dadurch gekennzeichnet ,**
**dass** das Pilotlicht (10) und das Polymerisationslicht (11) von einer gemeinsamen Lichtquelle (4, 25, 31) gespeist werden, wobei das Pilotlicht (10) durch eine niedrigere Lichtleistung der gemeinsamen Lichtquelle erzeugt wird.

6. Einrichtung nach einem der Patentansprüche 1 bis 5,
**dadurch gekennzeichnet,**
**dass** Pilotlicht (10) und Polymerisationslicht (11) einen gemeinsamen Lichtleiter (5, 26, 27,35) haben.

7. Einrichtung nach Patentanspruch 6, **dadurch gekennzeichnet , dass** der Lichtleiter (7) zur Einkopplung des reflektierten Pilotlichtes (10) an der Lichteintrittsstelle angeschliffen ist.

8. Einrichtung nach einem der Patentansprüche 1 bis 7 ,
**dadurch gekennzeichnet , dass** als Lichtleiter (5, 7, 27, 35) ein Glasfaserstab verwendet ist.

9. Einrichtung nach einem der Patentansprüche 1 bis 8 ,
**dadurch gekennzeichnet ,**
**dass** ein vom Timer (15) aktivierter akustischer und/oder optischer Signalgeber (16) vorgesehen ist, der das Ende der Polymerisationszeit anzeigt.

10. Einrichtung nach einem der Patentansprüche 1 bis 9 ,
**dadurch gekennzeichnet ,**
**dass** als Lichtleiter (5, 27, 35) für das Polymerisationslicht (11) ein an seinem freien Ende abgekröpfter Glasstab verwendet ist, der dreh- und abnehmbar an einem Halter angeordnet ist, und der Glasstab halterunsseitig kegelförmig angeschliffen ist, und der Lichtsensor (8, 29) korrespondierend zur Abstrahlfläche (30) diese Anschliffs angeordnet ist.

11. Einrichtung nach einem der Patentansprüche 1 bis 10,
**dadurch gekennzeichnet , dass** als Lichterzeuger für das Polymerisationslicht (11) und für das Pilotlicht (10) ein oder mehrere, auf den Lichtleiter (35) fokussierte Leuchtdioden (31) vorgesehen sind.

12. Einrichtung nach einem der Patentansprüche 1 bis 11,
**dadurch gekennzeichnet ,**
**dass** als Lichtsensor (8, 29) für das reflektierte Pilötlicht (10) eine Empfänger-Diode (32) vorgesehen ist.

13. Einrichtung nach Patentanspruch 11 oder 12, da- durch **gekennzeichnet** , dass die Dioden (31, 32) auf einem gemeinsamen Träger (33) angeordnet und vorzugsweise in ein das Licht fokussierendes Substrat (34) eingebettet sind.

14. Einrichtung nach einem der Patentansprüche 11 bis 13, **dadurch** gekennzeich- net, dass die Dioden (31, 32) einzeln oder in Gruppen beschaltet sind und so von der Steuerelektronik (13) individuell aktivierbar sind.

15. Einrichtung nach einem der Patentansprüche 1 bis 14,
**dadurch gekennzeichnet ,**
**dass** Mittel vorgesehen sind, die die spektrale Empfindlichkeit des Lichtsensors (8, 29), bzw. das auf diesen einfallende Licht auf vorzugsweise 400 bis 500 nm begrenzen.

## Claims

1. A system for the polymerization of photocuring plastics materials, particularly dental filling materials or adhesives, comprising at least one light source (4, 25, 31,) for generating, on the one hand, a polymerizing light (11) in a light spectrum suitable for polymerization and, on the other hand, a pilot light (10), further comprising a power supply source (12) for supplying power to said light source, an optical fiber system adjoining said light source and comprising optical fibers (5, 7, 26, 27, 35) adapted, on the one hand, to transfer the polymerizing light (11) and the pilot light (10) to the object (2) to be machined, and, on the other hand, for inputting the pilot light (10) reflected from said object (2) into an optical sensor (8, 29, 32), and further including an electronic control unit (13), in which the signals emitted by the optical sensor (8, 29, 32) are processed and which provides an activating signal for said light source (4, 25, 31) for generating the polymerizing light (11) and for a timer (15) adapted to regulate the duration of activation of said light source, **characterized in that** an amplifier (14) is provided for amplification of the signals emitted by said optical sensor (8, 29, 32) and that the response threshold of said amplifier (14) is adjusted such that said light source for generating the polymerizing light (11) is not activated until a predefined distance (a) has been reached.

2. A system as defined in claim 1, **characterized in that** said light source (4, 25, 31) for generating the polymerizing light (11) is periodically activated and deactivated and in the deactivation phases thereof said pilot light (10) is activated, the activation phases of the pilot light being very short compared with the activation phases of the polymerizing light.

3. A system as defined in claim 2, **characterized in that** in the deactivation phases of the polymerizing light (11) said timer (15) is deactivated and is only reactivated when said distance (a) has been reached.

4. A system as defined in claim 1, **characterized in that** the pilot light (10) is activated continuously during machining.

5. A system as defined in any one of claims 1 to 4, **characterized in that** said pilot light (10) and said polymerizing light (11) originate from a common light source (4, 25, 31), said pilot light (10) being generated by a lower optical output of said common light source.

6. A system as defined in any one of claims 1 to 5, **characterized in that** the pilot light (10) and the polymerizing light (11) have a common optical fiber (5, 26, 27, 35).

7. A system as defined in claim 6, **characterized in that** said optical fiber (7) for inputting the reflected pilot light (10) is ground at the point of light entry.

8. A system as defined in any one of claims 1 to 7, **characterized in that** the optical fiber (5, 7, 27, 35) used is a glass rod.

9. A system as defined in any one of claims 1 to 8, **characterized in that** there is provided an acoustic and/or optical signal generator ((16), which is activated by said timer (15) and which signals the termination of the polymerization period.

10. A system as defined in any one of claims 1 to 9, **characterized in that** the optical fiber (5, 27, 35) used for the polymerizing light (11) is a glass rod which is cranked at its free end and is disposed in a holder, in which it can be turned and from which it can be removed, and the glass rod is conically ground at its supported end while said optical sensor (8, 29) is disposed at an appropriate position relative to the light emitting surface (30) of the said ground face.

11. A system as defined in any one of claims 1 to 10, **characterized in that** said light generator for the polymerizing light (11) and for the pilot light (10) consists of one or more light-emitting diodes (31) focused onto said optical fiber (35).

12. A system as defined in any one of claims 1 to 11, **characterized in that** the optical sensor (8, 29) for the reflected pilot light (10) is a receiver diode (32).

13. A system as defined in claim 11 or claim 12, **characterized in that** said diodes (31, 32) are disposed on a common support (33) and are preferably embedded in a light focusing substrate (34).

14. A system as defined in any one of claims 11 to 13, **characterized in that** said diodes (31, 32) are connected individually or in groups and can thus be individually activated by the control electronics (13).

15. A system as defined in any one of claims 1 to 14, **characterized in that** means are provided which restrict the spectral responsivity of the optical sensor (8, 29), or of the light incident thereon, to preferably from 400 to 500 nm.

## Revendications

1. Dispositif de polymérisation de matériaux synthétiques photodurcissables, en particulier de matériaux de remplissage ou de collage dentaires, contenant au moins une source lumineuse (4, 25, 31) pour produire, d'une part, une lumière de polymérisation (11) dans un spectre lumineux convenant à la polymérisation et, d'autre part, une lumière pilote (10), une source d'alimentation en énergie (12) pour alimenter la source lumineuse, un système de guidage de lumière se raccordant à la source lumineuse avec des conducteurs optiques (5, 7, 26, 27, 35), d'une part, pour transférer la lumière de polymérisation (11) et la lumière pilote (10) sur l'objet à traiter (2) et, d'autre part, pour injecter la lumière pilote (10) réfléchie sur l'objet (2) dans un capteur de lumière (8, 29, 32), contenant en outre une unité de commande électronique (13), dans laquelle les signaux provenant du capteur de lumière (8, 29, 32) sont traités et qui donne un signal de démarrage à la source lumineuse (4, 25, 31) pour la lumière de polymérisation (11) et à une minuterie (15) déterminant la durée d'allumage de la source lumineuse, **caractérisé en ce qu'**il est prévu un amplificateur (14) qui amplifie les signaux provenant du capteur de lumière (8, 29, 32) et **en ce que** le seuil de réponse de l'amplificateur (14) est ajusté de sorte que la source lumineuse pour la lumière de polymérisation (11) ne soit allumée que lorsqu'une distance définie (a) est atteinte.

2. Dispositif selon la revendication 1, **caractérisé en ce que** la source lumineuse (4, 25, 31) pour la lumière de polymérisation (11) est périodiquement connectée et déconnectée et, dans les phases de déconnexion, la lumière pilote (10) est connectée, les phases de connexion pour la lumière pilote étant très courtes en comparaison des phases de connexion pour la lumière de polymérisation.

3. Dispositif selon la revendication 2, **caractérisé en ce que**, dans les phases de déconnexion de la lumière de polymérisation (11), la minuterie (15) est interrompue et n'est réactivée que lorsque la distance (a) est atteinte.

4. Dispositif selon la revendication 1, **caractérisé en ce que** la lumière pilote (10) est connectée en continu pendant le traitement d'un objet.

5. Dispositif selon l'une quelconque des revendications 1 à 4, **caractérisé en ce que** la lumière pilote (10) et la lumière de polymérisation (11) sont alimentées par une source lumineuse commune (4, 25, 31), la lumière pilote (10) étant produite par une puissance lumineuse moindre de la source lumineuse commune.

6. Dispositif selon l'une quelconque des revendications 1 à 5, **caractérisé en ce que** la lumière pilote (10) et la lumière de polymérisation (11) ont un conducteur optique commun (5, 26, 27, 35).

7. Dispositif selon la revendication 6, **caractérisé en ce que** le conducteur optique (7) est aminci au point d'entrée de la lumière pour l'injection de la lumière pilote réfléchie (10).

8. Dispositif selon l'une quelconque des revendications 1 à 7, **caractérisé en ce que** l'on utilise une tige de fibre de verre comme conducteur optique (5, 7, 27, 35).

9. Dispositif selon l'une quelconque des revendications 1 à 8, **caractérisé en ce qu'**il est prévu un émetteur de signal acoustique et/ou optique activé par la minuterie (15), ledit émetteur (16) affichant la fin de la période de polymérisation.

10. Dispositif selon l'une quelconque des revendications 1 à 9, **caractérisé en ce que** l'on utilise comme conducteur optique (5, 27, 35) pour la lumière de polymérisation (11) une tige de verre coudée à son extrémité libre et qui est montée sur un support de manière à pouvoir tourner et à être retirée, et la tige de verre est amincie en forme de cône du côté support et le capteur de lumière (8, 29) est agencé de manière à correspondre à la surface émettrice (30) de cet amincissement.

11. Dispositif selon l'une quelconque des revendications 1 à 10, **caractérisé en ce que** l'on prévoit comme générateur de lumière pour la lumière de polymérisation (11) et pour la lumière pilote (10) une ou plusieurs diodes électroluminescentes (31) focalisées sur le conducteur optique (35).

12. Dispositif selon l'une quelconque des revendications 1 à 11, **caractérisé en ce que** l'on prévoit une diode réceptrice (32) comme capteur de lumière (8, 29) pour la lumière pilote réfléchie (10).

13. Dispositif selon la revendication 11 ou 12, **caractérisé en ce que** les diodes (31, 32) sont agencées sur un support commun (33) et de préférence intégrées à un substrat (34) focalisant la lumière.

14. Dispositif selon l'une quelconque des revendications 11 à 13, **caractérisé en ce que** les diodes (31, 32) sont câblées individuellement ou en groupes et peuvent donc être activées individuellement par le système électronique de commande (13).

15. Dispositif selon l'une quelconque des revendications 1 à 14, **caractérisé en ce qu'**il est prévu des moyens qui limitent la sensibilité spectrale du capteur de lumière (8, 29) ou selon le cas la lumière incidente sur celui-ci, de préférence, à 400 à 500 nm.
